Europäisches Patentamt

European Patent Office

Office européen des brevets

(11) Veröffentlichungsnummer: **0 464 463 A1**

## EUROPÄISCHE PATENTANMELDUNG

(21) Anmeldenummer: **91110013.9**

(51) Int. Cl.5: **A61B 17/28**, A61B 17/02

(22) Anmeldetag: **19.06.91**

(30) Priorität: **03.07.90 DE 4021153**

(43) Veröffentlichungstag der Anmeldung: **08.01.92 Patentblatt 92/02**

(84) Benannte Vertragsstaaten:
**AT BE DE DK ES FR GB IT NL**

(71) Anmelder: **Richard Wolf GmbH**
**Pforzheimer Strasse 32**
**W-7134 Knittlingen(DE)**

(72) Erfinder: **Hiltebrandt, Siegfried**
**August-Lämmle-Strasse 18**
**W-7134 Knittlingen(DE)**
Erfinder: **Wolf, Johann**
**Nikolaus-Müller-Strasse 13**
**W-7518 Bretten(DE)**

(74) Vertreter: **Vollmann, Heiko, Dipl.-Ing. et al**
**Patentanwälte Wilcken & Wilcken,**
**Musterbahn 1**
**W-2400 Lübeck 1(DE)**

(54) **Organmanipulator.**

(57) Es ist ein Organmanipulator (1) zum Freilegen eines in einer Körperhöhle eines Lebewesens befindlichen zu untersuchenden oder zu therapierenden Organs beschrieben, das einen Spreizkörper (8) aus einem Mehrgelenk-Hebelsystem aus schwenkbeweglich miteinander verbundenen Gelenkarmen (9,10,11,12) aufweist, die aus einer gestreckten Lage zum Einführen in die Körperhöhle in eine gespreizte Lage überführbar sind, wobei die Gelenkarme (9,10,11,12) in ein dreieckförmiges Flächengebilde überführbar sind.

FIG. 3

Die Erfindung geht aus von einem medizinischen Instrument zum Manipulieren von körperinneren Organen von Lebewesen, mit einem Spreizkörper, der am distalen Ende einer in einem Schaft geführten Stange angeordnet ist, die an ihrem proximalen Ende mit einer Handhabe versehen und in dem Schaft axial verschiebbar geführt ist, um den Spreizkörper in eine gestreckte oder gespreizte Stellung zu bringen.

Endoskopische Eingriffe in der Bauchhöhle setzen zum einen eine mittels Körperhöhlengas aufgedehnte Körperhöhle und zum anderen den freien Zugang zu dem zu untersuchenden oder therapierenden Organ voraus. Ist ein freier Zugang zu einem bestimmten Organ nicht möglich, d. h. ist der zu untersuchende und zu therapierende Teil oder Bereich des Organs oder das gesamte Organ durch ein anderes Organ verlegt, so muß der betreffende Organbereich freigelegt werden.

Das Freilegen bzw. die Manipulation des zu behandelnden Organs erfolgt hierbei entsprechend dem Stand der Technik mit Fasszangen oder, wie in der DE-PS 37 09 706 beschrieben, mittels drei fingerartig ausgebildeter, federelastischer Elemente, die an ihren distalen, d. h. unmittelbar an dem zu manipulierenden Organ oder dergleichen angreifenden Enden Kugelelemente aufweisen, um Verletzungen zu vermeiden.

Nachteilig bei den Manipulatoren nach dem Stand der Technik ist, daß diese aufgrund ungenügender Kraftübertragung nur eine unzureichende Manipulation ermöglichen und zumindest teilweise auch Verletzungen verursachen können. Speziell bei dem Manipulator nach der DE-PS 37 09 706 besteht aufgrund der relativ geringen mechanischen Stabilität die Gefahr, daß sich die einzelnen Finger durch die angreifende Kraft verbiegen. Die in den Außenschaft einziehbaren Fingerelemente führen durch eine Verbiegung beim Ausfahren aus dem Schaft eine unkontrollierte Eigenbewegung aus, wodurch die Verwendbarkeit erheblich eingeschränkt wird. Ein weiterer Nachteil dieses Manipulators besteht darin, daß sich die Spreizkraft der Spreizelemente aus der Federvorspannung derselben herleitet, die Spreizkraft also unbeeinflußbar jeweils in ihrem Maximum festgelegt ist. Das kann dazu führen, daß die Spreizkraft nicht ausreicht, so daß unbemerkt ein Gleichgewichtszustand zwischen Spreizkraft und Spreizwiderstand entsteht mit dem Ergebnis, daß die Begrenzung des Spreizweges nicht durch den Schaft des Instrumentes erfolgt, sondern durch den Spreizwiderstand, so daß die Spreizkraft dann unkontrolliert das manipulierte Organ belastet. Das kann zu Schäden an diesem führen.

Diese dem Stand der Technik anhaftenden Nachteile sollen durch den Erfindungsgegenstand vermieden werden,und dieser soll ermöglichen, daß zum einen relativ hohe Kräfte bei der Organmanipulation ausgeübt werden können und zum anderen vermieden wird, daß evtl. auf unsachgemäße Handhabung zurückzuführende Beschädigungen an dem Manipulator zu Verletzungen an dem Organ oder dergleichen führen.

Diese Aufgabe wird erfindungsgemäß durch die kennzeichnenden Teile des Patentanspruches 1 gelöst. Vorteilhafte Ausgestaltungen der Erfindung sind den Unteransprüchen entnehmbar.

Mit Hilfe eines solchen Instrumentes ist es möglich, ein zu manipulierendes Organ unter definierter Krafteinwirkung beiseite zu drängen, wobei der Spreizweg des Spreizkörpers aufgrund der direkten und zwangsläufigen Umsetzung der Längsbewegung der Stange in die Spreizbewegung eindeutig steuerbar und die Spreizstellung statisch fixierbar ist.

Die Erfindung wird nachstehend anhand eines in der Zeichnung dargestellten Ausführungsbeispiels näher erläutert. Es zeigen:

Figur 1    eine Seitenansicht des Manipulators mit gestrecktem Spreizkörper;

Figur 2    eine Seitenansicht nach Figur 1 mit dem Spreizkörper in teilweise gespreizter Stellung;

Figur 3    eine Seitenansicht nach Figur 1 mit voll gespreiztem Spreizkörper.

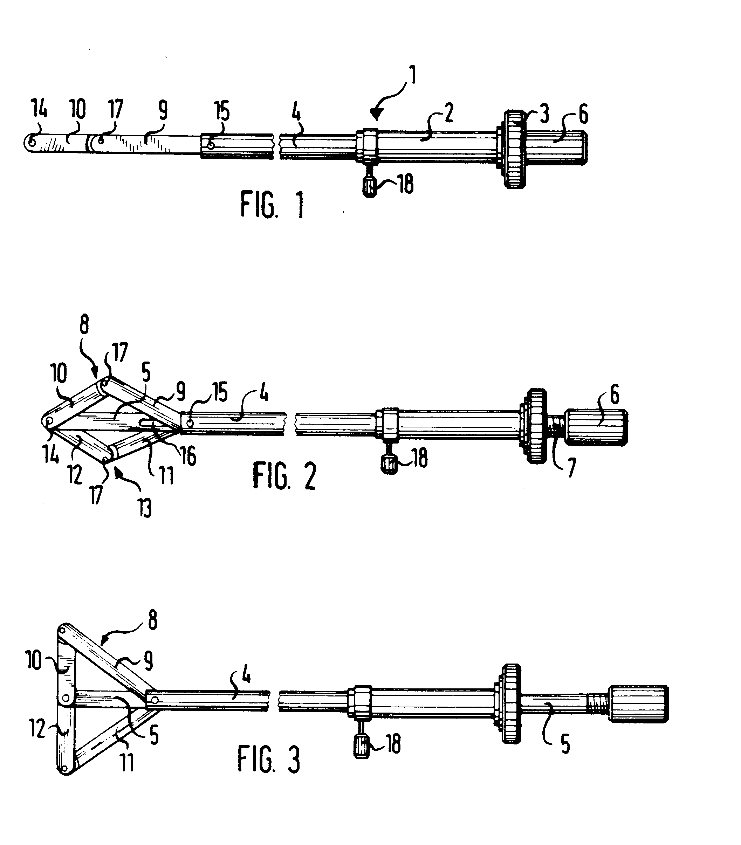

Wie aus den Figuren 1 bis 3 ersichtlich, besteht der erfindungsgemäße Manipulator 1 aus einem rohrförmigen Handhabenteil 2 mit einem radial drehbaren und axial gesicherten Schraubring 3 an seinem proximalen Ende. Der Handhabenteil 2 ist distalseitig mittels eines rohrförmigen Schaftes 4 verlängert. In dem Schaft 4 ist eine Stange 5 verschiebbar gelagert, die sich auch durch den Handhabenteil 2 hindurch erstreckt und an ihrem proximalen Ende mit einer Handhabe 6 und einem vor diesem angeordneten Gewinde 7 versehen ist. Die Stange 5 ragt aus dem distalen Ende des Schaftes 4 heraus und trägt im Bereich ihres distalen Endes einen Spreizkörper 8. Dieser besteht aus einem Mehrgelenk-Hebelsystem aus schwenkbeweglich miteinander verbundenen vier Gelenkarmen 9, 10, 11 und 12. Jeweils zwei dieser Gelenkarme 9, 10 oder 11, 12 sind durch ein Mittelgelenk 13 miteinander verbunden und einander gegenüberliegenden Seiten der Stange 5 zugeordnet. Dabei ist jeweils das eine Ende der beiden Gelenkpaare 9, 10 bzw. 11, 12 mit dem distalen Ende der Stange 5 und das andere Ende mit dem distalen Ende des Schaftes 4 mittels Gelenkstiften 14 bzw. 15 gelenkig verbunden, wobei letztere die Stange 5 im Bereich eines in dieser angeordneten Längsschlitzes 16 durchgreift und in dem Schaft 4 festgelegt ist. Die Mittelgelenke 13 werden durch Gelenkstifte 17 gebildet, die in der gestreckten Lage der Gelenkarme 9, 10 bzw. 11, 12 jenseits der Ebene der Mittelachse der Stange 5 gelegen sind.

Zur Einführung des Manipulators, beispielsweise durch eine Trokarhülse hindurch, wird der Spreizkörper in die gestreckte Stellung gebracht. Das geschieht dadurch, daß das Gewinde 7 im Bereich des proximalen Endes der Stange 5 durch Drehen des Schraubringes 3 in diesen hineingezogen und dadurch die Stange 5 aus dem distalen Ende des Schaftes 4 herausgeschoben wird. Das Einschieben kann nun beispielsweise unter Beobachtung durch ein anderwärts in die betreffende Körperhöhle eingebrachtes Endoskop erfolgen. Nach gewünschter Plazierung an oder neben dem zu manipulierenden Organ kann nun durch entgegengesetze Drehung des Schraubringes 3 die Stange 5 in den Schaft 4 hineingezogen werden mit der Wirkung, daß sich der Abstand der Gelenkstifte 14 und 15 zueinander verkürzt. Die Folge ist ein Ausscheren der Mittelgelenke 13, was dadurch zustandekommt, daß die Gelenkstifte 17 in der Strecklage der Gelenkarmpaare 9, 10 bzw. 11, 12 nicht in der Ebene der Mittelachse der Stange 5 liegen, so daß die Einzugbewegung derselben eine die Mittelgelenke 13 ausstellende Komponente erzeugt. Die radial nach außen gerichtete Spreizwirkung wird zusätzlich dadurch verstärkt, daß an den beiden Enden der Gelenkarme 9, 11 jeweils ein Biegefederelement (nicht dargestellt) mit seinem einen Ende festgelegt ist, während sich das gegenüberliegende andere Ende an der Innenwand des Schaftes 4, lose anliegend, abstützt. Die über das Gewinde 7 übertragene Zugbewegung kann dann durch Außereingriffkommen des Gewindes 7 und des Schraubringes 3 durch Herausziehen der Stange 5 fortgesetzt werden, bis der Gelenkstift 15 nahezu am distalen Ende des Längsschlitzes 16 zur Anlage kommt, welche Stellung annähernd die maximale Spreizstellung des Spreizkörpers 8 ergibt. Durch entgegengesetzes Drehen des Schraubringes 3 wird ein auf der Stange 5 sich im Abstand zum Gewinde 7 befindliches weiteres Gewinde (nicht dargestellt) in diesen hineingezogen, was bewirkt, daß der Spreizkörper 8 in seine maximale Spreizstellung gebracht und in dieser fixiert wird.

Durch den sich am distalen Ende des Handhabenteiles 3 befindlichen Schlauchanschluß 18 kann in den Innenraum des Manipulators 1 eine Spülflüssigkeit zur Beseitigung eventueller Verunreinigungen eingeleitet und im Anschluß daran die Sterilisation des Manipulators vorgenommen werden. Ferner besteht die Möglichkeit der Gaszuführung in eine Körperhöhle.

Um bei gleichzeitiger Anwendung eines Lasers und einer Video-Übertragungseinrichtung eine durch störende Reflexionen verursachte Bildbeeinträchtigung zu vermeiden und um eine hohe Absorption und Streuung der Laserstrahlung zu erreichen, ist die Oberfläche des gesamten in die Körperhöhle eingeführten Instrumententeiles mit einem schwarzen Überzug versehen, der keine glatte Oberfläche aufweist.

## Patentansprüche

1. Medizinisches Manipulatorinstrument zum Manipulieren von körperinneren Organen von Lebewesen, mit einem Spreizkörper, der am distalen Ende einer in einem Schaft geführten Stange angeordnet ist, die an ihrem proximalen Ende mit einer Handhabe versehen und in dem Schaft axial verschiebbar geführt ist, um den Spreizkörper in eine gestreckte oder gespreizte Stellung zu bringen, dadurch gekennzeichnet, daß der Spreizkörper (8) ein Mehrgelenk-Hebelsystem aus schwenkbeweglichen, miteinander verbundenen Gelenkarme (9,10,11,12) aufweist, die aus einer gestreckten Stellung in eine Spreizstellung bringbar sind.

2. Manipulator nach Anspruch 1, dadurch gekennzeichnet, daß das Mehrgelenk-Hebelsystem vier Gelenkarme (9, 10, 11, 12) umfaßt, wobei jeweils zwei derselben durch ein Mittelgelenk (13) miteinander verbunden und einander gegenüberliegenden Seiten der in den Schaft (4) geführten Stange (5) zugeordnet sind und jeweils das eine Ende der beiden Gelenkarmpaare (9, 10, 11, 12) mit dem distalen Ende der Stange (5) und das andere Ende mit dem distalen Ende des Schaftes (4) gelenkig verbunden sind.

3. Manipulator nach Anspruch 2, dadurch gekennzeichnet, daß die gelenkigen Verbindungen Gelenkstifte (14, 15, 17) aufweisen, wobei der im Bereich des distalen Endes des Schaftes (4) angeordnete Gelenkstift (15) die Stange (5) im Bereich eines in dieser befindlichen Längsschlitzes (16) durchgreift und in dem Schaft (4) festgelegt ist.

4. Manipulator nach einem der Ansprüche 1 bis 3, dadurch gekennzeichnet, daß die Gelenkstifte (14, 15) im Bereich der distalen Enden der Stange (5) bzw. des Schaftes (4) in der Ebene der Mittelachse der Stange (5) und die die Mittelgelenke (13) bildenden Gelenkstifte (17) in der gestreckten Lage der Gelenkarme (9, 10, 11, 12) außerhalb der Ebene der Mittelachse der Stange (5) angeordnet sind.

5. Manipulator nach einem der Ansprüche 1 bis 4, dadurch gekennzeichnet, daß die mit dem distalen Ende der Stange (5) gelenkig verbundenen Gelenkarme (10, 12) als Ausstellenker für die Gelenkarme (9, 11) fungieren, wobei die Gelenkarme (10, 12) in der maximalen Spreizstellung im wesentlichen miteinander fluchten.

FIG. 1

FIG. 2

FIG. 3

# Europäisches Patentamt

## EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | US-A-2 655 154 (RICHTER)<br>* Spalte 1, Zeile 39 - Spalte 3, Zeile 13; Figuren 1,2,4 *<br>– – – | 1,2,4,5 | A 61 B 17/28<br>A 61 B 17/02 |
| A | FR-A-2 588 746 BLAGOVESCHENSKY GOSUDARSTVEN-NY MEDITSINSKY INSTITUT ET VSESOJUZNY NAUCHNO-ISSLEDOVATELSKY I ISPYTATELNY INSTITUT MEDITSINSKOI TEKHNIKI<br>* Seite 3, Zeile 12 - Seite 4, Zeile 8; Anspruch; Figuren 1,2 *<br>– – – | 1,2,4 | |
| A | US-A-3 750 652 (SHERWIN)<br>* Spalte 2, Zeilen 43-67; Spalte 3, Zeilen 24-40; Figuren 1-4 *<br>– – – | 1,2 | |
| A | US-A-1 947 649 (KADAVY)<br>* Seite 1, Zeilen 40-78; Figuren 1,2 *<br>– – – – – | 1 | |

RECHERCHIERTE SACHGEBIETE (Int. Cl.5)

A 61 B
A 61 M

Der vorliegende Recherchenbericht wurde für alle Patentansprüche erstellt

| Recherchenort | Abschlußdatum der Recherche | Prüfer |
|---|---|---|
| Berlin | 23 September 91 | MONNE E.M.B. |